# EUROPEAN PATENT APPLICATION

(11) **EP 2 957 911 A1**
(43) Date of publication of application: **23.12.2015**
(21) Application number: 14752156.1
(22) Date of filing: 17.02.2014
(51) Int. Cl.: G01N 33/53, C07K 16/06, C07K 17/04, C12N 15/09, C12P 21/08, G01N 33/543

(54) **INDOXYL SULFATE MEASUREMENT METHOD**

(30) Priority: 15.02.2013 JP 2013027628
(71) Applicant: Kureha Corporation, Chuo-ku Tokyo 103-8552 (JP)
(72) Inventor: HOSHI Hirotaka, Tokyo 103-8552 (JP); KIKUCHI Kaori, Tokyo 103-8552 (JP); ITOH Yoshiharu, Tokyo 103-8552 (JP); KONAGAI Ayano, Tokyo 103-8552 (JP)
(74) Representative: Zimmermann & Partner Patentanwälte mbB
(86) International application number: PCT/JP2014/053584
(87) International publication number: WO 2014/126230

(57) **Abstract**

An object of the present invention is to provide a competitive EIA method capable of quantitatively measuring indoxyl sulfate in blood. The problem described above can be solved by an indoxyl sulfate measurement method based on a competitive method comprising: (1) a step in which albumin in a specimen is pretreated; (2) a specimen-antibody reaction step in which the pretreated specimen and anti-indoxyl sulfate antibodies are brought into contact with each other in the substantial absence of exogenous mammalian albumin; (3) an immobilized IS-antibody reaction step in which the specimen-antibody reaction solution is brought into contact, in the substantial absence of exogenous mammalian albumin, with an immobilized insoluble support in which indoxyl sulfate is immobilized and then blocked with a buffer solution containing substantially no mammalian albumin; and (4) a step in which anti-indoxyl sulfate antibodies which have bonded to the immobilized insoluble support are detected.

SEQUENCE LISTING

<110>  KUREHA CORPORATION

<120>  Method for measuring indoxyl sulfate

<130>  KRH-933

<150>  JP 2013-027628
<151>  2013-02-15

<160>  4

<170>  PatentIn version 3.1

<210>  1
<211>  417
<212>  DNA
<213>  Mus musculus

<400>  1
gaattcatgg aatgcagctg ggttattctc ttcatcctgt caggaattgc aggtgtccac      60

tcccaggttc agctgcagca gtctggacct gagctggtga agccaggggc ttcagtgaag     120

atgtcctgca aggcttctgg atacacattc actgactatg ttataatctg ggtgaagcag     180

agacctggac agggccttga gtggattgga gagatttatc ctggaagtac tggtacttac     240

cagaatgaga agttcaaggg caaggccaca ctgacagcag acagatcctc caacacagcc     300

tacatgcagc tcagtagcct gacatctgag gactctgcgg tctatttctg tgcaagagga     360

gggtataata actggtttgc tttctggggc caagggactc tggtcactgt ctctgca        417


<210>  2
<211>  139
<212>  PRT
<213>  Mus musculus

<400>  2

Glu Phe Met Glu Cys Ser Trp Val Ile Leu Phe Ile Leu Ser Gly Ile
1               5                   10                  15


Ala Gly Val His Ser Gln Val Gln Leu Gln Gln Ser Gly Pro Glu Leu
            20                  25                  30


Val Lys Pro Gly Ala Ser Val Lys Met Ser Cys Lys Ala Ser Gly Tyr
        35                  40                  45


Thr Phe Thr Asp Tyr Val Ile Ile Trp Val Lys Gln Arg Pro Gly Gln
    50                  55                  60


Gly Leu Glu Trp Ile Gly Glu Ile Tyr Pro Gly Ser Thr Gly Thr Tyr
65                  70                  75                  80

Gln Asn Glu Lys Phe Lys Gly Lys Ala Thr Leu Thr Ala Asp Arg Ser
                85                  90                  95

Ser Asn Thr Ala Tyr Met Gln Leu Ser Ser Leu Thr Ser Glu Asp Ser
            100                 105                 110

Ala Val Tyr Phe Cys Ala Arg Gly Gly Tyr Asn Asn Trp Phe Ala Phe
        115                 120                 125

Trp Gly Gln Gly Thr Leu Val Thr Val Ser Ala
    130                 135

<210>  3
<211>  390
<212>  DNA
<213>  Mus musculus

<400>  3
gaattcatgg agacagacac actcctgcta tgggtactgc tgctctgggt tccaggttcc      60

actggtgaca ttgtgctgac acagtctcct gcttccttag ctgtatctct ggggcagagg     120

gccaccatct catacagggc cagcaaaagt gtcagtacat ctggctatag ttatatgcac     180

tggaaccaac agaaaccagg acagccaccc agactcctca tctatcttgt atccaaccta     240

gaatctgggg tccctgccag gttcagtggc agtgggtctg ggacagactt caccctcaac     300

atccatcctg tggaggagga ggatgctgca acctattact gtcagcacat tagggagctt     360

acacgttcgg aggggggacc aagctggaaa                                      390

<210>  4
<211>  130
<212>  PRT
<213>  Mus musculus

<400>  4

Glu Phe Met Glu Thr Asp Thr Leu Leu Leu Trp Val Leu Leu Leu Trp
1               5                   10                  15

Val Pro Gly Ser Thr Gly Asp Ile Val Leu Thr Gln Ser Pro Ala Ser
            20                  25                  30

Leu Ala Val Ser Leu Gly Gln Arg Ala Thr Ile Ser Tyr Arg Ala Ser
        35                  40                  45

Lys Ser Val Ser Thr Ser Gly Tyr Ser Tyr Met His Trp Asn Gln Gln
    50                  55                  60

Lys Pro Gly Gln Pro Pro Arg Leu Leu Ile Tyr Leu Val Ser Asn Leu
65                  70                  75                  80

Glu Ser Gly Val Pro Ala Arg Phe Ser Gly Ser Gly Ser Gly Thr Asp
                85                  90                  95

Phe Thr Leu Asn Ile His Pro Val Glu Glu Glu Asp Ala Ala Thr Tyr
            100                 105                 110

Tyr Cys Gln His Ile Arg Glu Leu Thr Arg Ser Glu Gly Gly Pro Ser
        115                 120                 125

Trp Lys
    130

## Description

### TECHNICAL FIELD

The present invention relates to an indoxyl sulfate measurement method. With the present invention, indoxyl sulfate can be measured with high sensitivity.

### BACKGROUND ART

Uremia is a disease in which renal function deteriorates acutely or chronically. As one cause of the onset of uremia, there are substances that are considered to be uremic toxins, and indoxyl sulfate is one such substance (Non-Patent Document 1). In fact, it has been reported that serum indoxyl sulfate in chronic renal failure patients is increased substantially by approximately 60 times that of healthy subjects (Non-Patent Document 2). Accordingly, measuring the uremic toxin concentration in body fluids - in serum, in particular - is extremely useful for the diagnosis or testing of the disease, and this measurement method can be further applied to the assessment of therapeutic effects, prognosis prediction, dietary therapy markers, and the like.

In this indoxyl sulfate measurement method, analysis was conventionally performed by gas chromatography or high-performance liquid chromatography. In Patent Document 1, a competitive enzyme immunoassay method (also called a "competitive EIA method" hereafter) using antibodies is disclosed as a method of simply measuring indoxyl sulfate.

### CITATION LIST

### Patent Literature

Patent Document 1: Japanese Unexamined Patent Application Publication No. H10-265457A

### Non-Patent Document

Non-Patent Document 1: "Seminars in Nephrology", 1996 (Holland), Vol. 16, p. 167-182
Non-Patent Document 2: "Journal of the Japanese Society for Dialysis Therapy", 1988 (Japan), Vol. 21, p. 951-956 (1988)

### SUMMARY OF INVENTION

### Technical Problem

When the present inventors performed the competitive EIA method using antibodies described in Patent Document 1, it was possible to quantitatively measure indoxyl sulfate in urine, but it became clear that it is difficult to quantitatively measure indoxyl sulfate in blood. Therefore, an object of the present invention is to provide a competitive EIA method capable of quantitatively measuring indoxyl sulfate in blood.

### Solution to Problem

As a result of conducting dedicated research on competitive EIA methods capable of quantitatively measuring indoxyl sulfate in blood, the present inventors surprisingly discovered that albumin in human blood inhibits the binding of indoxyl sulfate and anti-indoxyl sulfate antibodies. The present inventors further discovered that bovine serum albumin (also called "BSA" hereafter) ordinarily used for blocking in competitive EIA inhibits the binding of indoxyl sulfate and anti-indoxyl sulfate antibodies. Further, the present inventors discovered that BSA contained in a reaction solution of a competitive EIA method inhibits the binding of indoxyl sulfate and anti-indoxyl sulfate antibodies. Inhibition by these albumins was marked for low-concentration indoxyl sulfate. In Patent Document 1, indoxyl sulfate in human serum is measured, and there are a plurality of commercially available ELISA kits for measuring indoxyl sulfate in serum or plasma. Accordingly, it was unexpected to those having ordinary skill in the art that albumin in blood in a specimen, BSA in a blocking solution or reaction buffer, or the like inhibits the binding of indoxyl sulfate and anti-indoxyl sulfate antibodies. The present inventors discovered that indoxyl sulfate in blood can be accurately measured by pretreating albumin in a specimen, by blocking an indoxyl sulfate-immobilized insoluble support with a Tris buffer solution not containing albumin, and by reacting indoxyl sulfate and anti-indoxyl sulfate antibodies using a Tris buffer solution not containing albumin. Further, the present inventors discovered that indoxyl sulfate in blood can be accurately measured by blocking an anti-indoxyl sulfate antibody-immobilized insoluble support with a buffer solution not containing exogenous mammalian albumin and by reacting an anti-indoxyl sulfate antibody-immobilized insoluble support with indoxyl sulfate (specimen and labeled indoxyl sulfate) using a buffer solution not containing exogenous mammalian albumin. The present invention is based on such knowledge.

Therefore, the present invention relates to the following.
[1] An indoxyl sulfate measurement method based on a competitive method comprising: (1) a step in which albumin in a specimen is pretreated; (2) a specimen-antibody reaction step in which the pretreated specimen and anti-indoxyl sulfate antibodies are brought into contact with each other in the substantial absence of exogenous mammalian albumin; (3) an immobilized IS-antibody reaction step in which the specimen-antibody reaction solution is brought into contact, in the substantial absence of exogenous mammalian albumin, with an immobilized insoluble support in which indoxyl sulfate is immobilized and then blocked with a buffer solution containing substantially no mammalian albumin; and (4) a step in which anti-indoxyl sulfate antibodies which have bonded to the immobilized insoluble support are detected;
[2] an indoxyl sulfate measurement method based on a competitive method comprising: (1) a step in which albumin in a specimen is pretreated; (2) a specimen-antigen reaction step in which the pretreated specimen and labeled indoxyl sulfate are brought into contact with each other in the substantial absence of exogenous mammalian albumin; (3) an immobilized antibody-labeled IS reaction step in which the specimen-antigen reaction solution is brought into contact, in the substantial absence of exogenous mammalian albumin, with an immobilized insoluble support in which anti-indoxyl sulfate antibodies are immobilized and then blocked with a buffer solution containing substantially no mammalian albumin; and (4) a step in which labeled indoxyl sulfate which has bonded to the immobilized insoluble support is detected;
[3] an indoxyl sulfate measurement method based on a competitive method comprising: (1) a step in which albumin in a specimen is pretreated; (2) a specimen-antigen reaction step in which the pretreated specimen and labeled indoxyl sulfate are brought into contact with each other in the substantial absence of exogenous mammalian albumin; (3) an immobilized antibody-labeled IS reaction step in which the specimen-antigen reaction solution is brought into contact, in the substantial absence of exogenous mammalian albumin, with an immobilized insoluble support and anti-indoxyl sulfate antibodies in which antibodies against anti-indoxyl sulfate antibodies are immobilized and then blocked with a buffer solution containing substantially no mammalian albumin; and (4) a step in which labeled indoxyl sulfate which has bonded to the immobilized insoluble support is detected;
[4] an indoxyl sulfate measurement method based on a competitive method comprising: (1) a step in which albumin in a specimen is pretreated; (2) a specimen-antigen reaction step in which the pretreated specimen is brought into contact with labeled indoxyl sulfate and anti-indoxyl sulfate antibodies in the substantial absence of exogenous mammalian albumin; (3) an immobilized antibody-labeled IS reaction step in which the specimen-antigen reaction solution is brought into contact, in the substantial absence of exogenous mammalian albumin, with an immobilized insoluble support in which antibodies against anti-indoxyl sulfate antibodies are immobilized and then blocked with a buffer solution containing substantially no mammalian albumin; and (4) a step in which labeled indoxyl sulfate which has bonded to the immobilized insoluble support is detected;
[5] the indoxyl sulfate measurement method according to one of [1] to [4], wherein the anti-indoxyl sulfate antibodies are antibodies containing a light chain complementarity determining region comprising an amino acid sequence represented by SEQ ID NO: 2 and a heavy chain complementarity determining region comprising an amino acid sequence represented by SEQ ID NO: 4;
[6] the indoxyl sulfate measurement method according to one of [1] to [5], wherein the pretreatment of albumin is treatment with a pretreatment solution or deproteinization treatment;
[7] the indoxyl sulfate measurement method according to one of [1], [5], and [6], wherein the specimen-antibody reaction step (2) and the immobilized IS-antibody reaction step (3) are performed simultaneously;
[8] the indoxyl sulfate measurement method according to one of [2] to [6], wherein the specimen-antigen reaction step (2) and the immobilized antibody-labeled IS reaction step (3) are performed simultaneously;
[9] an immobilized insoluble support in which indoxyl sulfate is immobilized and then blocked with a buffer solution containing substantially no mammalian albumin;
[10] an immobilized insoluble support in which anti-indoxyl sulfate antibodies are immobilized and then blocked with a buffer solution containing substantially no mammalian albumin;
[11] an indoxyl sulfate measurement kit including (1) the immobilized insoluble support according to [9] or the immobilized insoluble support according to [10] and (2) a pretreatment solution for pretreating albumin in a specimen or a reagent for removing albumin in a specimen by deproteinization;
[12] an anti-indoxyl sulfate antibody containing a light chain complementarity determining region comprising an amino acid sequence represented by SEQ ID NO: 2 and a heavy chain complementarity determining region comprising an amino acid sequence represented by SEQ ID NO: 4;
[13] use of one or more selected from the group consisting of (1) anti-indoxyl sulfate antibodies, (2) an immobilized insoluble support in which indoxyl sulfate is immobilized and then blocked with a buffer solution containing no albumin, and (3) a pretreatment solution for pretreating albumin in a specimen and/or a reagent for removing albumin in a specimen by deproteinization in the production of a uremia diagnostic kit; or
[14] use of one or more selected from the group consisting of (1) labeled indoxyl sulfate, (2) an immobilized insoluble support in which anti-indoxyl sulfate antibodies are immobilized and then blocked with a buffer solution containing no albumin, and (3) a pretreatment solution for pretreating albumin in a specimen and/or a reagent for removing albumin in a specimen by deproteinization in the production of a uremia diagnostic kit.

### Advantageous Effects of Invention

With the indoxyl sulfate measurement method of the present invention, it is possible to quantitatively measure indoxyl sulfate in blood. In addition, the sensitivity of a competitive EIA method can be further increased by pretreating albumin in a specimen by means of deproteinization. Further, the sensitivity of a competitive EIA method can be further increased by using antibodies containing a light chain complementarity determining region comprising an amino acid sequence represented by SEQ ID NO: 2 and a heavy chain complementarity determining region comprising an amino acid sequence represented by SEQ ID NO: 4.

### Brief Description of Drawing

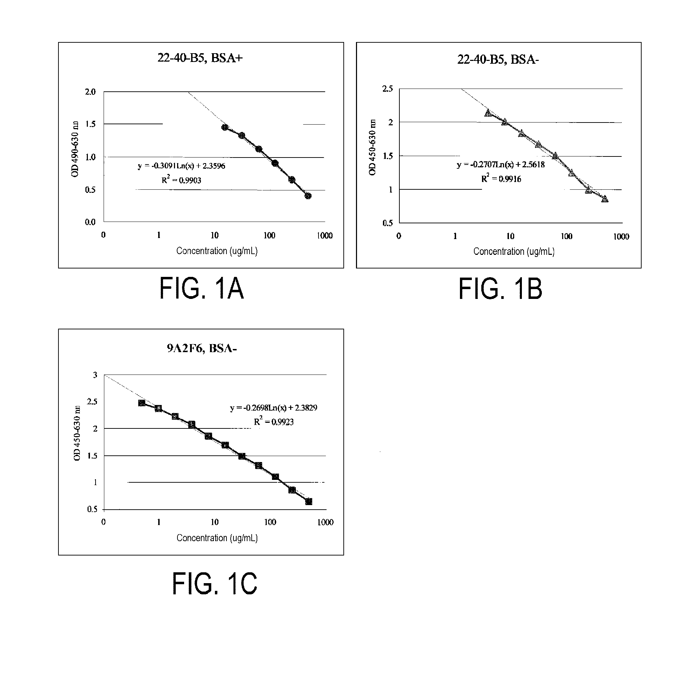

FIGS. 1A to 1C are graphs illustrating calibration curves in cases in which BSA is added to a reaction buffer solution using two types of monoclonal antibodies and a case in which BSA is not added. (A): 22-40-B5 (BSA+); (B) 22-40-B5 (BSA-); (C) 9A2F6 (BSA-)

### Description of Embodiments

### [1] Indoxyl sulfate measurement method

The indoxyl sulfate measurement method of the present invention is an immunoassay method based on a competitive method using anti-indoxyl sulfate antibodies, wherein the concentration of indoxyl sulfate in a specimen is measured by inducing the competition of indoxyl sulfate in a specimen and indoxyl sulfate of a known concentration (also called "competitive indoxyl sulfate" hereafter) in bonds with anti-indoxyl sulfate antibodies.

Examples of this competitive method include a method of detecting anti-indoxyl sulfate antibodies binding to immobilized competitive indoxyl sulfate (also called an "antigen immobilization method" hereafter) and a method of detecting competitive indoxyl sulfate binding to immobilized anti-indoxyl sulfate antibodies (also called an "antibody immobilization method" hereafter).

### [1-1] Antigen immobilization method

The indoxyl sulfate measurement method based on the antigen immobilization method of the present invention comprises: (1) a step in which albumin in a specimen is pretreated; (2) a specimen-antibody reaction step in which the pretreated specimen and anti-indoxyl sulfate antibodies are brought into contact with each other in the substantial absence of exogenous mammalian albumin; (3) an immobilized IS-antibody reaction step in which the specimen-antibody reaction solution is brought into contact, in the substantial absence of exogenous mammalian albumin, with an immobilized insoluble support in which indoxyl sulfate is immobilized and then blocked with a buffer solution containing substantially no mammalian albumin; and (4) a step in which anti-indoxyl sulfate antibodies which have bonded to the immobilized insoluble support are detected.

In this specification, "exogenous mammalian albumin" refers to albumin other than albumin originally contained in the measured specimen. For example, when human serum is used as a specimen, this includes mammalian albumin other than human albumin - that is, bovine albumin, equine albumin, rat albumin, mouse albumin, pig albumin, sheep albumin, or goat albumin.

In addition, in this specification, "in the substantial absence of exogenous mammalian albumin" means albumin is not present in an amount at which the effects of the present invention are not sufficiently achieved. That is, this means that mammalian albumin is not present in an amount that would substantially inhibit the binding of indoxyl sulfate and anti-indoxyl sulfate antibodies. Accordingly, mammalian albumin exhibiting at most 10% inhibition of measurement values may be present in comparison to measurement values in cases in which absolutely no mammalian albumin is present.

Further, in this specification, a "buffer solution containing substantially no mammalian albumin" means that the buffer solution does not contain mammalian albumin in an amount at which the effects of the present invention are not sufficiently achieved. That is, this means that mammalian albumin is not contained in an amount that would substantially inhibit the binding of indoxyl sulfate and anti-indoxyl sulfate antibodies. Accordingly, mammalian albumin exhibiting at most 10% inhibition of measurement values may be present in comparison to measurement values in cases in which absolutely no mammalian albumin is present, for example.

### 〈〈Pretreatment step (1)〉〉

The pretreatment step (1) is a step for freeing indoxyl sulfate in a specimen from albumin in the specimen so as to facilitate binding with anti-indoxyl sulfate antibodies by pretreating the albumin in the specimen.

The method of pretreatment is not limited as long as the binding of indoxyl sulfate and anti-indoxyl sulfate antibodies in the specimen is in an accelerated state, but examples include treatment with a pretreatment solution or deproteinization treatment, and deproteinization treatment is particularly preferable. This is because when proteins in the reaction solution are removed, the inhibition of reactions due to proteins other than albumin is also eliminated.

### (Treatment with a pretreatment solution)

The pretreatment solution is not limited as long as it weakens bonds between indoxyl sulfate and albumin in the specimen and accelerates the binding of indoxyl sulfate and anti-indoxyl sulfate antibodies. For example, a buffer solution containing a surfactant may be used. The surfactant is not limited, and examples include nonionic surfactants, anionic surfactants, cationic surfactants, and/or amphoteric ion surfactants. Nonionic surfactants that do not inhibit antigen/antibody reactions are preferable, and Triton X, Tween, or Pluronic is particularly preferable.

In addition, the concentration of the surfactant is not particularly limited but is preferably from 0.002 to 2 wt.%, more preferably from 0.008 to 0.5 wt.%, and most preferably from 0.02 to 0.2 wt.%.

### (Deproteinization)

Deproteinization treatment is not limited as long as proteins including albumin can be substantially removed without removing the indoxyl sulfate in a specimen. In this specification, "substantially removing proteins including albumin" does not only mean that proteins are completely removed from a specimen, but also means that albumin is removed to an extent that indoxyl sulfate measurements can be performed with good reproducibility in the indoxyl sulfate measurement method of the present invention.

A known method can be used as the method of deproteinization treatment. For example, a method used in HPLC pretreatment can be used. Examples thereof include a method of modifying, insolubilizing, and removing proteins, a method of physically removing proteins by utilizing differences in molecule size, or an affinity method, but a method of modifying, insolubilizing, and removing proteins is preferable. This is because treatment in which proteins are insolubilized also has the effect of dissociating bonds between indoxyl sulfate and albumin. A commercially available kit may also be used for deproteinization treatment. For example, a Sirocco Protein Precipitation Plate may also be used. When this plate is used, 200 µL of acetonitrile is added to the plate, and 50 µL of the specimen is then added. The proteins in the specimen are precipitated by the acetonitrile. The precipitated proteins are filtered by means of aspiration from below the plate. The filtrate from which proteins are removed can be recovered and used as a pretreated specimen in the specimen-antibody reaction step (2).

### (Specimen)

The specimens that can be used in the indoxyl sulfate measurement method of the present invention are not particularly limited as long as they are specimens that may contain indoxyl sulfate, and examples thereof include blood, serum, plasma, lymphatic fluid, tissue fluid, saliva, urine, tears, sweat, milk, or tissue extract. The measurement method of the present invention can effectively measure specimens with high albumin content (for example, blood, serum, plasma, lymphatic fluid, tissue fluid, or tissue extract) and can also measure specimens with low albumin content (for example, saliva, urine, tears, milk, or sweat). In addition, the animal species from which specimens are acquired are also not limited, and specimens derived from humans, dogs, cats, cows, horses, rats, mice, pigs, sheep, or goats, for example, can be measured with the indoxyl sulfate measurement method of the present invention.

### <Specimen-antibody reaction step (2)>

The specimen-antibody reaction step (2) in the antigen immobilization method of the present invention is a step in which the pretreated specimen and anti-indoxyl sulfate antibodies are brought into contact with each other in the substantial absence of exogenous mammalian albumin.

In this specification, "brought into contact in the substantial absence of exogenous mammalian albumin" means that substantially no external mammalian albumin other than that contained in the specimen is added to the reaction buffer solution. That is, in this step, this means that the reaction buffer solution for diluting the anti-indoxyl sulfate antibodies does not contain external mammalian albumin in an amount at which the effect of the present invention is not sufficiently achieved. In other words, mammalian albumin may be contained in an amount at which the effect of the present invention is achieved. Here, when human serum is used as a specimen, for example, albumin is removed when the pretreated specimen is subjected to deproteinization, so the mixture of the pretreated specimen and anti-indoxyl sulfate antibodies contains substantially no albumin. On the other hand, when the pretreated specimen is subjected to treatment with a pretreatment solution but is not subjected to deproteinization, albumin is not removed, so the pretreated specimen and anti-indoxyl sulfate antibodies come into contact with each other in the presence of albumin. However, since external mammalian albumin is not added to the reaction buffer solution for diluting the anti-indoxyl sulfate antibodies, the pretreated specimen and anti-indoxyl sulfate antibodies come into contact with each other in the absence of exogenous mammalian albumin.

### (Reaction buffer solution)

The reaction buffer solution used in the specimen-antibody reaction step (2) does not contain mammalian albumin. The reaction buffer solution may contain proteins other than mammalian albumin, but a reaction buffer solution containing no proteins is preferable from the perspective of eliminating interactions between these proteins and antibodies or indoxyl sulfate. The buffer solution serving as a base is not particularly limited, and examples include a tris buffer solution, phosphate buffered saline (PBS), a MES buffer solution, a MOPS buffer solution, a MOPSO buffer solution, a HEPES buffer solution, a TES buffer solution, a Tricine buffer solution, a Bis-Tris buffer solution, an ADA buffer solution, an ACES buffer solution, a PIPES buffer solution, a BES buffer solution, a DIPSO buffer solution, a TAPSO buffer solution, a POPSO buffer solution, a HEPPS buffer solution, a HEPPSO buffer solution, a Bicine buffer solution, a TAPS buffer solution, a CHES buffer solution, a CAPS buffer solution, an Imidazole-HCl buffer solution, a Glycylglycine buffer solution, or a Glycinamide buffer solution. A buffer solution containing a compound having an amide group is preferable, and a tris buffer solution is particularly preferable. This is because a buffer solution containing a compound having an amide group can also be suitably used for blocking. The tris buffer solution is not particularly limited, and examples include a tris HCl buffer solution, a TE buffer solution, a TAE buffer solution, a TBE buffer solution, or tris buffered saline. The concentration of each of these buffer solutions is not particularly limited but is preferably from 5 to 200 mM, more preferably from 10 to 150 mM, and even more preferably from 10 to 100 mM.

The reaction buffer solution can be used to dilute anti-indoxyl sulfate antibodies in the specimen-antibody reaction step (2).

### (Surfactant)

The reaction buffer solution may contain a surfactant. The surfactant is not particularly limited, and examples include nonionic surfactants, anionic surfactants, cationic surfactants, and/or amphoteric ion surfactants. Nonionic surfactants that do not inhibit antigen/antibody reactions are preferable, and Triton X, Tween, or Pluronic is particularly preferable.

In addition, the concentration of the surfactant is not particularly limited but is preferably from 0.002 to 2 wt.%, more preferably from 0.008 to 0.5 wt.%, and most preferably from 0.02 to 0.2 wt.%.

### (Mammalian albumin)

In this specification, "mammalian albumin" refers to a group of proteins called albumin in mammals. Specifically, mammalian albumin is a protein typically produced by the liver and, in the case of mammals, accounts for 50 to 65% of proteins in serum. In addition, mammalian albumin is also contained in body fluids other than serum, and albumin contained in milk, for example, may also be called milk albumin.

In order to suppress nonspecific reactions of antibodies in the reaction buffer solution in an immunoassay method using antibodies, a protein such as bovine serum albumin is ordinarily added. The reaction buffer solution used in the present invention does not contain exogenous mammalian albumin (for example, serum albumin or milk albumin). This is because mammalian albumin inhibits the measurement of indoxyl sulfate in the indoxyl sulfate measurement method of the present invention. The origin of mammalian albumin not contained in the reaction buffer solution is not limited, and the reaction buffer solution does not contain albumin of any mammals, but specifically, the reaction buffer solution does not contain bovine albumin, human albumin, equine albumin, rat albumin, mouse albumin, pig albumin, sheep albumin, and goat albumin.

The reaction buffer solution may also contain proteins other than albumin (proteins with no affinity to indoxyl sulfate).

### (Anti-indoxyl sulfate antibodies)

The anti-indoxyl sulfate antibodies used in the indoxyl sulfate measurement method of the present invention are not limited as long as they can bind to indoxyl sulfate, and the antibodies may be monoclonal antibodies or polyclonal antibodies. However, from the perspective of specificity, monoclonal antibodies are preferable, and 9A2F6 monoclonal antibodies containing a light chain complementarity determining region comprising an amino acid sequence represented by SEQ ID NO: 2 and a heavy chain complementarity determining region comprising an amino acid sequence represented by SEQ ID NO: 4 are particularly preferable.

The anti-indoxyl sulfate antibodies used in the indoxyl sulfate measurement method can be produced by a known method with the exception that a protein-bound linker-providing indoxyl sulfate is used as an immunogen. As described above, the anti-indoxyl sulfate antibodies may be polyclonal antibodies or monoclonal antibodies, but monoclonal antibodies are preferable. In addition, existing or commercially available antibodies may also be used. Monoclonal antibodies can be produced in accordance with the method of Koehler and Milstein (Nature 256:495-497, 1975). In addition, polyclonal antibodies can be produced by periodically intracutaneously immunizing a rabbit with an antigen alone or after binding with BSA, KLA, or the like and mixing with an adjuvant such as Freund's complete adjuvant, collecting blood at the point when the antibody titer in blood increases, and then using this directly as an antiserum or using the antibodies after purification by a known method.

In addition, an antibody fragment containing an antigen binding site for indoxyl sulfate may also be used as an anti-indoxyl sulfate antibody. Examples of antibody fragments include F(ab')₂, Fab', Fab, or Fv, diabodies, single-chain antibody molecules, and multispecific antibodies formed from antibody fragments. These antibody fragments can be obtained by digesting antibodies with proteolytic enzymes (for example, pepsin, papain, or the like) using a conventional method and then purifying the proteins with a conventional protein separation and purification method, or the antibody fragments can be prepared by genetic recombination.

The volume of the pretreated specimen and anti-indoxyl sulfate antibodies in the specimen-antibody reaction step (2) is not particularly limited, but the step may be performed at approximately 20 µL to 500 µL, for example. In addition, the ratio of the pretreated specimen and anti-indoxyl sulfate antibodies is not limited, but the substances may be mixed and brought into contact with each other at a ratio of approximately 1:10 to 10:1. The concentration of the anti-indoxyl sulfate antibodies may also be determined as needed and is not particularly limited, but a concentration of from 0.01 µg/mL to 100 µg/mL may be used, and the concentration is preferably from 0.1 µg/mL to 10 µg/mL and more preferably from 0.5 µg/mL to 5 µg/mL. The contact time of the pretreated specimen and anti-indoxyl sulfate antibodies is also not particularly limited, and the substances can be brought into contact with each other from between 30 minutes and one day/night, but the contact time is preferably from 30 minutes to 2 hours. In addition, the contact temperature is also not particularly limited, and contact can be performed at approximately 4°C to 40°C, but the contact temperature is preferably from 30 to 37°C.

### <Immobilized IS-antibody reaction step (3)>

The immobilized IS-antibody reaction step (3) in the antigen immobilization method of the present invention is a step in which the specimen-antibody reaction solution is brought into contact, in the substantial absence of exogenous mammalian albumin, with an immobilized insoluble support in which indoxyl sulfate is immobilized and then blocked with a buffer solution containing substantially no mammalian albumin.

In this step, the specimen-antibody reaction solution obtained in the specimen-antibody reaction step (2) is brought into contact with an insoluble support in which indoxyl sulfate is immobilized in the absence of exogenous mammalian albumin. In this specification, as in the specimen-antibody reaction step (2), "brought into contact in the substantial absence of exogenous mammalian albumin" means that substantially no external mammalian albumin other than that contained in the specimen is added to the reaction buffer solution. That is, this means that the reaction buffer solution for diluting the anti-indoxyl sulfate antibodies used in the specimen-antibody reaction step (2) does not contain external mammalian albumin in an amount at which the effect of the present invention is not sufficiently achieved. In other words, mammalian albumin may be contained in an amount at which the effect of the present invention is achieved. In addition, in this step, when the specimen-antibody reaction solution obtained in the specimen-antibody reaction step (2) is added to an immobilized insoluble support and a reaction buffer solution is further added, a reaction buffer solution not containing any external mammalian albumin is used. The reaction buffer solution used in this step may be any of the reaction buffer solutions listed in the "Specimen-antibody reaction step (2)" section described above.

### <Immobilized insoluble support>

The immobilized insoluble support used in this step is one in which indoxyl sulfate is immobilized and then blocked with a buffer solution not containing albumin and is preferably an immobilized insoluble support which is blocked with a Tris buffer solution not containing albumin. The insoluble support that is used is not particularly limited as long as it is one that is used in ordinary immunoassay methods, and examples include immunoassay microplates, beads, or magnetic particles. The material of the insoluble support is also not particularly limited, and examples include polymers such as polyethylene, polystyrene, polypropylene, polyvinyl chloride, polyester, polyacrylonitrile, fluororesins, crosslinked dextran, and polysaccharide, as well as nitrocelluose, paper, agarose, and combinations thereof. More specifically, a 96-well or 384-well immunoassay microplate can be used.

### (Indoxyl sulfate)

The indoxyl sulfate used for immobilization is not limited, but a substance bound to a carrier protein is preferable. The carrier protein is not particularly limited as long as it is a protein other than albumin, and examples include keyhole limpet hemacyanin, ovalbumin, or transferrin, thyroglobulin, or immunoglobulin, but transferrin is preferable. The method of binding to the carrier protein is also in accordance with an ordinary method. For example, a bound substance of indoxyl sulfate and a carrier protein can be obtained by mixing indoxyl sulfate and a protein in a buffer solution of pH 6 to 8 or water, reacting the mixture for a few hours to approximately 20 hours at room temperature, performing dialysis, and then centrifuging the internal fluid to remove impurities.

The buffer solution for dissolving indoxyl sulfate is also not particularly limited, and examples include a tris buffer solution, phosphate buffered saline (PBS), a MES buffer solution, a MOPS buffer solution, a MOPSO buffer solution, a HEPES buffer solution, a TES buffer solution, a Tricine buffer solution, a Bis-Tris buffer solution, an ADA buffer solution, an ACES buffer solution, a PIPES buffer solution, a BES buffer solution, a DIPSO buffer solution, a TAPSO buffer solution, a POPSO buffer solution, a HEPPS buffer solution, a HEPPSO buffer solution, a Bicine buffer solution, a TAPS buffer solution, a CHES buffer solution, a CAPS buffer solution, an Imidazole-HCl buffer solution, a Glycylglycine buffer solution, or a Glycinamide buffer solution. A tris buffer solution is particularly preferable.

Immobilization may also be performed in accordance with the conditions for normally immobilizing an antigen to an insoluble support in an immunoassay method, but in the case of a 96-well plate, indoxyl sulfate bound to a carrier protein with a concentration of 100 ng to 10 µg/mL can be immobilized at 4°C and O/N at a volume of 50 µL to 200 µL/well.

### (Blocking solution)

The blocking solution does not contain mammalian albumin. The blocking solution may contain proteins other than mammalian albumin, but a blocking solution containing no proteins is preferable from the perspective of eliminating interactions between these proteins and antibodies or indoxyl sulfate. The buffer solution serving as a base is not limited as long as it does not contain mammalian albumin, and examples include a tris buffer solution, phosphate buffered saline (PBS), a MES buffer solution, a MOPS buffer solution, a MOPSO buffer solution, a HEPES buffer solution, a TES buffer solution, a Tricine buffer solution, a Bis-Tris buffer solution, an ADA buffer solution, an ACES buffer solution, a PIPES buffer solution, a BES buffer solution, a DIPSO buffer solution, a TAPSO buffer solution, a POPSO buffer solution, a HEPPS buffer solution, a HEPPSO buffer solution, a Bicine buffer solution, a TAPS buffer solution, a CHES buffer solution, a CAPS buffer solution, an Imidazole-HCl buffer solution, a Glycylglycine buffer solution, or a Glycinamide buffer solution. However, in order to perform effective blocking, a buffer solution containing a compound having an amide group is preferable, and a tris buffer solution is particularly preferable. The tris buffer solution is not particularly limited, and examples include a tris HCl buffer solution, a TE buffer solution, a TAE buffer solution, a TBE buffer solution, or tris buffered saline.

In addition, the blocking solution may contain a surfactant. The surfactant is not particularly limited, and examples include nonionic surfactants, anionic surfactants, cationic surfactants, and/or amphoteric ion surfactants. Nonionic surfactants that do not inhibit antigen/antibody reactions are preferable, and Triton X, Tween, or Pluronic is particularly preferable.

In addition, the concentration of the surfactant is not particularly limited but is preferably from 0.002 to 2 wt.%, more preferably from 0.008 to 0.5 wt.%, and most preferably from 0.02 to 0.2 wt.%.

For example, when a 96-well plate is used as an insoluble support, the amount of the blocking solution that is added should be greater than the volume of the buffer solution containing indoxyl sulfate used for immobilization, and blocking may be performed, for example, at a volume of 200 µL to 400 µL. In addition, the blocking temperature and time are also not particularly limited, and blocking may be performed, for example, for 30 minutes to one day/night at 4°C to 37°C.

### <Detection step (4)>

The detection step (4) in the antigen immobilization method of the present invention is a step in which anti-indoxyl sulfate antibodies which have bonded to the indoxyl sulfate immobilized on the immobilized insoluble support are detected. The detection of anti-indoxyl sulfate antibodies may be performed by directly labeling the anti-indoxyl sulfate antibodies with a labeling substance and then detecting the antibodies as a signal thereof. That is, one aspect of the detection step (4) is (a) a step in which a mark of labeled anti-indoxyl sulfate antibodies is detected and, specifically, a step in which a signal of labeled anti-indoxyl sulfate antibodies (primary antibodies) which have bonded to the immobilized insoluble support is detected (one-step method, direct method). Further, the detection of anti-indoxyl sulfate antibodies may be performed by labeling secondary antibodies (for example, anti-mouse immunoglobulin antibodies) against anti-indoxyl sulfate antibodies, binding the labeled secondary antibodies to the anti-indoxyl sulfate antibodies, and detecting the marking of the labeled secondary antibodies as a signal. That is, another aspect of the detection step (4) is (b) a step in which a mark of labeled antibodies against anti-indoxyl sulfate antibodies is detected and, specifically, a step in which labeled secondary antibodies against anti-indoxyl sulfate antibodies are brought into contact, and a signal of the labeled secondary antibodies which have bonded to the immobilized insoluble support is detected (two-step method, indirect method).

### (Labeling substance and reaction substrate)

Examples of antibody labeling substances include horseradish peroxidase (HRP), alkaline phosphatase, β-galactosidase, and luciferase. In addition to enzymes, luminescent substances such as acridinium derivatives, fluorescent substances such as rhodamine, fluorescein isothiacyanate (FITC), or europium, radioactive substances such as ³H, ¹⁴C, or I¹²⁵, or the like can also be used as labeling substances. In addition, the substrate or luminescent derivative substance can be selected appropriately in accordance with the labeling substance. Further, the antibodies can be labeled with biotin, and the avidin bound to biotin can be labeled with the aforementioned labeling substance and detected as a signal.

### (Reaction buffer solution for detection)

The reaction buffer solution for detection used in the detection step (4) is used to dilute the labeled secondary antibodies against the anti-indoxyl sulfate antibodies in the two-step method. The reaction buffer solution for detection is not particularly limited but is preferably a solution not containing mammalian albumin. The reaction buffer solution for detection may contain proteins other than mammalian albumin, but a reaction buffer solution for detection containing no proteins is preferable from the perspective of eliminating interactions between these proteins and antibodies or indoxyl sulfate. The buffer solution serving as a base is not limited, and examples include a tris buffer solution, phosphate buffered saline (PBS), a MES buffer solution, a MOPS buffer solution, a MOPSO buffer solution, a HEPES buffer solution, a TES buffer solution, a Tricine buffer solution, a Bis-Tris buffer solution, an ADA buffer solution, an ACES buffer solution, a PIPES buffer solution, a BES buffer solution, a DIPSO buffer solution, a TAPSO buffer solution, a POPSO buffer solution, a HEPPS buffer solution, a HEPPSO buffer solution, a Bicine buffer solution, a TAPS buffer solution, a CHES buffer solution, a CAPS buffer solution, an Imidazole-HCl buffer solution, a Glycylglycine buffer solution, or a Glycinamide buffer solution. A buffer solution containing a compound having an amide group is preferable, and a tris buffer solution is particularly preferable. This is because a buffer solution containing a compound having an amide group can also be suitably used for blocking. The tris buffer solution is not particularly limited, and examples include a tris HCl buffer solution, a TE buffer solution, a TAE buffer solution, a TBE buffer solution, or tris buffered saline.

### (Labeled secondary antibody contact)

The labeled secondary antibodies are diluted with the reaction solution for detection, added to the insoluble support, and reacted. The reaction time is not particularly limited, and the reaction can be performed for 30 minutes to one day/night, but the reaction time is preferably from 30 minutes to 2 hours. In addition, the reaction temperature is also not particularly limited, and the reaction can be performed at approximately 4°C to 40°C, but the reaction temperature is preferably from 30 to 37°C.

### (Signal detection)

In the detection step (4), the anti-indoxyl sulfate antibodies which have bonded to the immobilized indoxyl sulfate can be detected by detecting a signal of a fluorescent substance, a radioactive substance, a substrate for an enzyme, a luminescent derivative, and the like.

In the indoxyl sulfate measurement method of the present invention, the indoxyl sulfate in the specimen and the immobilized competitive indoxyl sulfate compete in bonds with the anti-indoxyl sulfate antibodies, so when the amount of indoxyl sulfate in the specimen is large, the signal is low, whereas when the amount of indoxyl sulfate in the specimen is small, the signal is high.

### <Simultaneous implementation of the specimen-antibody reaction step (2) and the immobilized IS-antibody reaction step (3)>

In the indoxyl sulfate measurement method based on the antigen immobilization method of the present invention, the specimen-antibody reaction step (2) and the immobilized IS-antibody reaction step (3) can be performed simultaneously.

Specifically, this is a step in which the pretreated specimen, anti-indoxyl sulfate antibodies, and immobilized indoxyl sulfate are brought into contact with each other simultaneously on an immobilized insoluble support.

The reaction time is not particularly limited, and the reaction can be performed for 30 minutes to one day/night, but the reaction time is preferably from 30 minutes to 2 hours. In addition, the reaction temperature is also not particularly limited, and the reaction can be performed at approximately 4°C to 40°C, but the reaction temperature is preferably from 30 to 37°C.

In the indoxyl sulfate measurement method based on the antigen immobilization method of the present invention, the immobilized insoluble support can be washed between the respective steps.

### [1-2] Antigen immobilization method

The indoxyl sulfate measurement method based on the antigen immobilization method of the present invention comprises: (1) a step in which albumin in a specimen is pretreated; (2) a specimen-antigen reaction step in which the pretreated specimen and labeled indoxyl sulfate are brought into contact with each other in the substantial absence of exogenous mammalian albumin; (3) an immobilized antibody-labeled IS reaction step in which the specimen-antigen reaction solution is brought into contact, in the substantial absence of exogenous mammalian albumin, with an immobilized insoluble support in which anti-indoxyl sulfate antibodies are immobilized and then blocked with a buffer solution containing substantially no mammalian albumin; and (4) a step in which labeled indoxyl sulfate which has bonded to the immobilized insoluble support is detected.

In this specification, a "immobilized insoluble support in which anti-indoxyl sulfate antibodies are immobilized" includes not only cases in which anti-indoxyl sulfate antibodies are immobilized directly on an insoluble support, but also cases in which anti-indoxyl sulfate antibodies are immobilized on an insoluble carrier via a component with affinity to the anti-indoxyl sulfate antibodies (for example, antibodies against the anti-indoxyl sulfate antibodies) or cases in which anti-indoxyl sulfate antibodies are immobilized on an insoluble support via the bonding of avidin, biotin, and the like. Specifically, antibodies against the anti-indoxyl sulfate antibodies (for example, anti-mouse antibodies) may be immobilized on an insoluble support, and mouse anti-indoxyl sulfate antibodies may be immobilized on the insoluble support via these antibodies. In addition, avidin may be immobilized on an insoluble support, and biotin-labeled anti-indoxyl sulfate antibodies may be immobilized via the avidin.

In addition, the indoxyl sulfate measurement method based on the antigen immobilization method of the present invention comprises: (1) a step in which albumin in a specimen is pretreated; (2) a specimen-antigen reaction step in which the pretreated specimen and labeled indoxyl sulfate are brought into contact with each other in the substantial absence of exogenous mammalian albumin; (3) an immobilized antibody-labeled IS reaction step in which the specimen-antigen reaction solution is brought into contact, in the substantial absence of exogenous mammalian albumin, with an immobilized insoluble support and anti-indoxyl sulfate antibodies in which antibodies against anti-indoxyl sulfate antibodies are immobilized and then blocked with a buffer solution containing substantially no mammalian albumin; and (4) a step in which labeled indoxyl sulfate which has bonded to the immobilized insoluble support is detected.

Further, the indoxyl sulfate measurement method based on the antigen immobilization method of the present invention comprises: (1) a step in which albumin in a specimen is pretreated; (2) a specimen-antigen reaction step in which the pretreated specimen is brought into contact with labeled indoxyl sulfate and anti-indoxyl sulfate antibodies in the substantial absence of exogenous mammalian albumin; (3) an immobilized antibody-labeled IS reaction step in which the specimen-antigen reaction solution is brought into contact, in the substantial absence of exogenous mammalian albumin, with an immobilized insoluble support in which antibodies against anti-indoxyl sulfate antibodies are immobilized and then blocked with a buffer solution containing substantially no mammalian albumin; and (4) a step in which labeled indoxyl sulfate which has bonded to the immobilized insoluble support is detected.

### <Pretreatment step (1)>

The pretreatment step (1) in the antibody immobilization method can be conducted in the same way as the pretreatment step (1) in the antigen immobilization method.

### <Specimen-antigen reaction step (2)>

The specimen-antibody reaction step (2) in the antigen immobilization method is a step in which the pretreated specimen and anti-indoxyl sulfate antibodies are brought into contact with each other in the substantial absence of exogenous mammalian albumin. The buffer solution is not particularly limited and may be used without limitation to the "reaction buffer solution" described in the antibody immobilization method.

In this specification, "brought into contact in the substantial absence of exogenous mammalian albumin" means that substantially no external mammalian albumin other than the albumin contained in the specimen is added to the reaction buffer solution. That is, in this step, this means that the reaction buffer solution for diluting the labeled indoxyl sulfate does not contain external mammalian albumin in an amount at which the effect of the present invention is not sufficiently achieved. In other words, mammalian albumin may be contained in an amount at which the effect of the present invention is achieved. Here, when human serum is used as a specimen, for example, human serum albumin is removed when the pretreated specimen is subjected to deproteinization, so the mixture of the pretreated specimen and labeled indoxyl sulfate contains substantially no albumin. On the other hand, when the pretreated specimen is subjected to treatment with a pretreatment solution but is not subjected to deproteinization, human serum albumin is not removed, so the pretreated specimen and labeled indoxyl sulfate come into contact with each other in the presence of albumin. However, since external albumin is not added to the reaction buffer solution for diluting the labeled indoxyl sulfate, the pretreated specimen and the labeled indoxyl sulfate come into contact with each other in the absence of exogenous albumin.

The buffer solution used in this step may not contain proteins, but it preferably contains proteins other than mammalian albumin. The proteins other than mammalian albumin are not limited, and gelatin, casein, ovalbumin, commercially available blocking agents, or the like, for example, can be used.

The "reaction buffer solution", "surfactant", and "mammalian albumin" described in the antibody immobilization method are the same as the "reaction buffer solution", "surfactant", and "mammalian albumin" described in the "Specimen-antibody reaction step (2)" of the aforementioned antigen immobilization method.

### (Labeled indoxyl sulfate)

The labeled indoxyl sulfate used in the indoxyl sulfate measurement method based on the antibody immobilization method of the present invention is not limited as long as it binds to anti-indoxyl sulfate antibodies, and it may be, for example, free indoxyl sulfate or a substance bound to a carrier protein. The carrier protein is not particularly limited as long as it is a protein other than mammalian albumin (protein with no affinity to indoxyl sulfate), and examples include transferrin, keyhole limpet hemacyanin, thyroglobulin, ovalbumin, or immunoglobulin. The method of binding to the carrier protein is also in accordance with an ordinary method.

### (Labeling substance)

Examples of the labeling substance used for the labeled indoxyl sulfate include horseradish peroxidase (HRP), alkaline phosphatase, β-galactosidase, and luciferase. In addition to enzymes, luminescent substances such as acridinium derivatives, fluorescent substances such as rhodamine, fluorescein isothiacyanate (FITC), or europium, radioactive substances such as ³H, ¹⁴C, or I¹²⁵, or the like can also be used as labeling substances.

The volume of the pretreated specimen and the labeled indoxyl sulfate in the specimen-antibody reaction step (2) is not particularly limited, but the step may be performed at approximately 20 µL to 500 µL, for example. In addition, the ratio of the pretreated specimen and the labeled indoxyl sulfate is not limited, but the substances may be mixed and brought into contact with each other at a ratio of approximately 1:10 to 10:1. The concentration of the labeled indoxyl sulfate may also be determined as needed and is not particularly limited, but a concentration of from 0.01 µg/mL to 100 µg/mL may be used, and the concentration is preferably from 0.1 µg/mL to 10 µg/mL and more preferably from 0.5 µg/mL to 5 µg/mL.
The contact time of the pretreated specimen and anti-indoxyl sulfate antibodies is also not particularly limited, and the substances can be brought into contact with each other from between 30 minutes and one day/night, but the contact time is preferably from 30 minutes to 2 hours. In addition, the contact temperature is also not particularly limited, and contact can be performed at approximately 4°C to 40°C, but the contact temperature is preferably from 30 to 37°C.

### <Immobilized antibody-labeled IS reaction step (3)>

The immobilized antibody-labeled IS reaction step (3) is a step in which the specimen-antibody reaction solution is brought into contact, in the substantial absence of exogenous mammalian albumin, with an immobilized insoluble support in which anti-indoxyl sulfate antibodies are immobilized and then blocked with a buffer solution containing substantially no mammalian albumin. The buffer solution is not particularly limited and may be used without limitation to the "reaction buffer solution" described in the antibody immobilization method. In this step, the specimen-antigen reaction solution obtained in the specimen-antigen reaction step (2) described above is brought into contact with an insoluble support in which anti-indoxyl sulfate antibodies are immobilized in the substantial absence of exogenous mammalian albumin. In this specification, as in the specimen-antigen reaction step (2), "brought into contact in the substantial absence of exogenous mammalian albumin" means that substantially no external mammalian albumin is added to the reaction buffer solution. That is, this means that the reaction buffer solution for diluting the labeled indoxyl sulfate used in the specimen-antigen reaction step (2) does not contain external mammalian albumin in an amount at which the effect of the present invention is not sufficiently achieved. In addition, in this step, when the specimen-antigen reaction solution obtained in the specimen-antigen reaction step (2) is added to an immobilized insoluble support and a reaction buffer solution is further added, a reaction buffer solution not containing any external mammalian albumin is used.

The buffer solution used in this step may not contain proteins, but it preferably contains proteins other than mammalian albumin. The proteins other than mammalian albumin are not limited, and gelatin, casein, ovalbumin, commercially available blocking agents, or the like, for example, can be used.

The reaction buffer solution used in this step may be any of the reaction buffer solutions listed in the "Specimen-antibody reaction step (2)" section of the antigen immobilization method described above.

### <Immobilized insoluble support>

The immobilized insoluble support used in this step is an immobilized insoluble support in which anti-indoxyl sulfate antibodies are immobilized and then blocked with a buffer solution containing substantially no mammalian albumin. The buffer solution is not particularly limited and may be used without limitation to the "reaction buffer solution" described in the antibody immobilization method. The insoluble support that is used is not particularly limited as long as it is one that is used in ordinary immunoassay methods, and examples include immunoassay microplates, beads, or magnetic particles. The material of the insoluble support is also not particularly limited, and examples include polymers such as polyethylene, polystyrene, polypropylene, polyvinyl chloride, polyester, polyacrylonitrile, fluororesins, crosslinked dextran, and polysaccharide, as well as nitrocelluose, paper, agarose, and combinations thereof. More specifically, a 96-well or 384-well immunoassay microplate, for example, can be used.

### (Anti-indoxyl sulfate antibodies)

The anti-indoxyl sulfate antibodies that can be used for immobilization may be the anti-indoxyl sulfate antibodies listed in the "Specimen-antibody reaction step (2)" section of the antigen immobilization method described above.

The buffer solution for dissolving anti-indoxyl sulfate antibodies is also not particularly limited, and examples include a tris buffer solution, phosphate buffered saline (PBS), a MES buffer solution, a MOPS buffer solution, a MOPSO buffer solution, a HEPES buffer solution, a TES buffer solution, a Tricine buffer solution, a Bis-Tris buffer solution, an ADA buffer solution, an ACES buffer solution, a PIPES buffer solution, a BES buffer solution, a DIPSO buffer solution, a TAPSO buffer solution, a POPSO buffer solution, a HEPPS buffer solution, a HEPPSO buffer solution, a Bicine buffer solution, a TAPS buffer solution, a CHES buffer solution, a CAPS buffer solution, an Imidazole-HCl buffer solution, a Glycylglycine buffer solution, or a Glycinamide buffer solution. A tris buffer solution is particularly preferable.

Immobilization may also be performed in accordance with the conditions for normally immobilizing antigens to an insoluble support in an immunoassay method, but when a 96-well plate is used as an insoluble support, for example, anti-indoxyl sulfate antibodies with a concentration of 100 ng to 10 µg/mL can be immobilized at 4°C and O/N at a volume of 50 µL to 200 µL/well.

### (Blocking solution)

The blocking solution is one that does not contain mammalian albumin, but blocking can be performed in the same manner using the blocking solution described in the "Specimen-antibody reaction step (2)" section of the antigen immobilization method.

The buffer solution used in this step preferably contains proteins other than mammalian albumin. The proteins other than mammalian albumin are not limited, and gelatin, casein, ovalbumin, commercially available blocking agents, or the like, for example, can be used.

### <Detection step (4)>

The detection step (4) is a step in which labeled indoxyl sulfate which has bonded to the anti-indoxyl sulfate antibodies immobilized on the immobilized insoluble support are detected. The detection of the labeled indoxyl sulfate is performed by detecting the mark of labeled indoxyl sulfate as a signal. That is, one aspect of the detection step (4) is a step in which a mark of labeled indoxyl sulfate is detected and, specifically, a step in which a signal of labeled indoxyl sulfate which has bonded to the immobilized insoluble support is detected.

When a composition in which indoxyl sulfate and a support bind to one another is used, a signal can be detected by labeling the support or by using labeled antibodies against the support without directly labeling the indoxyl sulfate, but in this specification, such aspects are also included in the detection of labeled indoxyl sulfate.

In the detection step (4) of the antibody immobilization method of the present invention, the "labeling substance and reaction substrate" and the "reaction buffer solution for detection" listed in the "Detection step (4)" section of the antigen immobilization method described above can be used.

### (Signal detection)

In the detection step (4) of the antibody immobilization method of the present invention, the labeled indoxyl sulfate which has bonded to the immobilized anti-indoxyl sulfate antibodies can be detected by detecting a signal of a fluorescent substance, a radioactive substance, a substrate for an enzyme, a luminescent derivative, and the like.

In the indoxyl sulfate measurement method based on the antibody immobilization method of the present invention, the indoxyl sulfate in the specimen and the labeled indoxyl sulfate (competitive indoxyl sulfate) compete in bonds with the anti-indoxyl sulfate antibodies, so when the amount of indoxyl sulfate in the specimen is large, the signal is low, whereas when the amount of indoxyl sulfate in the specimen is small, the signal is high.

### <Simultaneous implementation of the specimen-antigen reaction step (2) and the immobilized antibody-labeled IS reaction step (3)>

In the indoxyl sulfate measurement method based on the antigen immobilization method of the present invention, the specimen-antigen reaction step (2) and the immobilized antibody-labeled IS reaction step (3) can be performed simultaneously.

Specifically, this is a step in which the pretreated specimen, labeled indoxyl sulfate, and immobilized anti-indoxyl sulfate antibodies are brought into contact with each other simultaneously on an immobilized insoluble support.

The reaction time is not particularly limited, and the reaction can be performed for 30 minutes to one day/night, but the reaction time is preferably from 30 minutes to 2 hours. In addition, the reaction temperature is also not particularly limited, and the reaction can be performed at approximately 4°C to 40°C, but the reaction temperature is preferably from 30 to 37°C.

In the indoxyl sulfate measurement method based on the antibody immobilization method of the present invention, the immobilized insoluble support can be washed between the respective steps.

The indoxyl sulfate measurement method of the present invention suppresses the effects of albumin, which inhibits the measurement of indoxyl sulfate, in a conventional indoxyl sulfate measurement method. Therefore, the present invention relates to a method for suppressing the inhibition of albumin measurements in an indoxyl sulfate measurement method based on a competitive method, the method comprising:
(1) a step in which albumin in a specimen is pretreated;
(2) a specimen-antibody reaction step in which the pretreated specimen and anti-indoxyl sulfate antibodies are brought into contact with each other in the substantial absence of exogenous mammalian albumin;
(3) an immobilized IS-antibody reaction step in which the specimen-antibody reaction solution is brought into contact, in the substantial absence of exogenous mammalian albumin, with an immobilized insoluble support in which indoxyl sulfate is immobilized and then blocked with a buffer solution containing substantially no mammalian albumin; and
(4) a step in which anti-indoxyl sulfate antibodies which have bonded to the immobilized insoluble support are detected.

In addition, the present invention relates to a method for suppressing the inhibition of albumin measurements in an indoxyl sulfate measurement method based on a competitive method, the method comprising:
(1) a step in which albumin in a specimen is pretreated;
(2) a specimen-antigen reaction step in which the pretreated specimen and labeled indoxyl sulfate are brought into contact with each other in the substantial absence of exogenous mammalian albumin;
(3) an immobilized antibody-labeled IS reaction step in which the specimen-antigen reaction solution is brought into contact, in the substantial absence of exogenous mammalian albumin, with an immobilized insoluble support in which anti-indoxyl sulfate antibodies are immobilized and then blocked with a buffer solution containing substantially no mammalian albumin; and
(4) a step in which labeled indoxyl sulfate which has bonded to the immobilized insoluble support is detected.

### [2] Uremia diagnostic method

The indoxyl sulfate measurement method of the present invention can be used as a uremia diagnostic method. The indoxyl sulfate measurement method of the present invention can also be used as an auxiliary uremia diagnostic method. In addition, the uremia diagnostic method of the present invention is performed by separating a specimen (for example, blood, serum, plasma, lymphatic fluid, tissue fluid, saliva, urine, tears, sweat, milk, or tissue extract) from inside the body and then measuring indoxyl sulfate in vitro. Further, the uremia diagnostic method of the present invention measures indoxyl sulfate in order to provide information necessary for the diagnosis of uremia and does not include a clinical assessment by a physician.

Examples of uremia in which indoxyl sulfate in the body increases include various renal disorders such as chronic renal failure and acute renal failure.

The anti-indoxyl sulfate antibodies described above can be used as antibodies for use in a uremia diagnosis (method).

### [3] Immobilized insoluble support

The immobilized insoluble support of the present invention is (A) an immobilized insoluble support in which indoxyl sulfate is immobilized and then blocked with a buffer solution containing no mammalian albumin, or (B) an immobilized insoluble support in which anti-indoxyl sulfate antibodies are immobilized and then blocked with a buffer solution containing no mammalian albumin.

The immobilized insoluble support (B) described above includes not only cases in which anti-indoxyl sulfate antibodies are immobilized directly on an insoluble support, but also cases in which anti-indoxyl sulfate antibodies are immobilized on an insoluble carrier via a component with affinity to the anti-indoxyl sulfate antibodies (for example, antibodies against the anti-indoxyl sulfate antibodies) or cases in which anti-indoxyl sulfate antibodies are immobilized on an insoluble support via the bonding of avidin, biotin, and the like. Specifically, antibodies against the anti-indoxyl sulfate antibodies (for example, anti-mouse antibodies) may be immobilized on an insoluble support, and mouse anti-indoxyl sulfate antibodies may be immobilized on the insoluble support via these antibodies. In addition, avidin may be immobilized on an insoluble support, and biotin-labeled anti-indoxyl sulfate antibodies may be immobilized via the avidin.

The immobilized insoluble support (A) described above can be used in the indoxyl sulfate measurement method based on the antigen immobilization method of the present invention, and the immobilized insoluble support (B) described above can be used in the indoxyl sulfate measurement method based on the antibody immobilization method of the present invention. The "indoxyl sulfate" and "blocking solution" used in the immobilized insoluble support (A) of the present invention maybe the "indoxyl sulfate" and "blocking solution" described in "[1] Indoxyl sulfate measurement method" above. The "anti-indoxyl sulfate antibodies" and "blocking solution" used in the immobilized insoluble support (A) of the present invention may be the "anti-indoxyl sulfate antibodies" and "blocking solution" described in "[1] Indoxyl sulfate measurement method" above. In addition, the insoluble support used in the immobilized insoluble support may use an insoluble support used in an ordinary immunoassay method without limitation, and examples include immunoassay microplates, beads, or magnetic particles. The material of the insoluble support is also not particularly limited, and examples include polymers such as polyethylene, polystyrene, polypropylene, polyvinyl chloride, polyester, polyacrylonitrile, fluororesins, crosslinked dextran, and polysaccharide, as well as nitrocelluose, paper, agarose, and combinations thereof. More specifically, a 96-well immunoassay microplate, a 384-well immunoassay microplate, and the like can be used.

### <Immobilized insoluble support (A) production method>

The production method for the immobilized insoluble support (A) comprises: (1) a step in which indoxyl sulfate is immobilized on an insoluble support; and (2) a step in which blocking is performed with a buffer solution containing substantially no mammalian albumin.

The immobilization step (1) is a step in which indoxyl sulfate is bound to the insoluble support by bringing a buffer solution containing indoxyl sulfate into contact with the insoluble support. The buffer solution for dissolving indoxyl sulfate is not particularly limited, and examples include a tris buffer solution, phosphate buffered saline (PBS), a MES buffer solution, a MOPS buffer solution, a MOPSO buffer solution, a HEPES buffer solution, a TES buffer solution, a Tricine buffer solution, a Bis-Tris buffer solution, an ADA buffer solution, an ACES buffer solution, a PIPES buffer solution, a BES buffer solution, a DIPSO buffer solution, a TAPSO buffer solution, a POPSO buffer solution, a HEPPS buffer solution, a HEPPSO buffer solution, a Bicine buffer solution, a TAPS buffer solution, a CHES buffer solution, a CAPS buffer solution, an Imidazole-HCl buffer solution, a Glycylglycine buffer solution, or a Glycinamide buffer solution. A tris buffer solution is particularly preferable. The tris buffer solution is not particularly limited, and examples include a tris HCl buffer solution, a TE buffer solution, a TAE buffer solution, a TBE buffer solution, or tris buffered saline. Indoxyl sulfate bound to a carrier protein (for example, transferrin-bound indoxyl sulfate) is dissolved in these buffer solutions to a concentration of 100 ng to 10 µg/mL and immobilized at 4°C and O/N at a volume of 50 µL to 200 µL/well.

The blocking step (2) is a step in which blocking is performed on the surface of the insoluble support by bringing a blocking solution into contact with the insoluble support in which indoxyl sulfate is immobilized. The blocking solution is not limited as long as it contains substantially no mammalian albumin, but the blocking solution preferably contains no proteins. Specific examples include a tris buffer solution, phosphate buffered saline (PBS), a MES buffer solution, a MOPS buffer solution, a MOPSO buffer solution, a HEPES buffer solution, a TES buffer solution, a Tricine buffer solution, a Bis-Tris buffer solution, an ADA buffer solution, an ACES buffer solution, a PIPES buffer solution, a BES buffer solution, a DIPSO buffer solution, a TAPSO buffer solution, a POPSO buffer solution, a HEPPS buffer solution, a HEPPSO buffer solution, a Bicine buffer solution, a TAPS buffer solution, a CHES buffer solution, a CAPS buffer solution, an Imidazole-HCl buffer solution, a Glycylglycine buffer solution, or a Glycinamide buffer solution. However, in order to perform effective blocking, a buffer solution containing a compound having an amide group is preferable, and a tris buffer solution is particularly preferable. The tris buffer solution is not particularly limited, and examples include a tris HCl buffer solution, a TE buffer solution, a TAE buffer solution, a TBE buffer solution, or tris buffered saline.
In addition, the blocking solution may contain a surfactant. The surfactant is not particularly limited, and examples include nonionic surfactants, anionic surfactants, cationic surfactants, and/or amphoteric ion surfactants. Nonionic surfactants that do not inhibit antigen/antibody reactions are preferable, and Triton X, Tween, or Pluronic is particularly preferable.
In addition, the concentration of the surfactant is not particularly limited but is preferably from 0.002 to 2 wt.%, more preferably from 0.008 to 0.5 wt.%, and most preferably from 0.02 to 0.2 wt.%.
In the case of a 96-well plate, for example, blocking can be performed by adding such a blocking solution in an amount of 50 µL to 300 µL and letting the mixture stand for between 10 minutes and one day/night at 4°C to 37°C, for example.

### <Immobilized insoluble support (B) production method>

The production method for the immobilized insoluble support (B) comprises: (1) a step in which anti-indoxyl sulfate antibodies are immobilized on an insoluble support; and (2) a step in which blocking is performed with a buffer solution containing substantially no mammalian albumin.

The immobilization step (1) is a step in which anti-indoxyl sulfate antibodies are bound to the insoluble support by bringing a buffer solution containing anti-indoxyl sulfate antibodies into contact with the insoluble support. The buffer solution for dissolving anti-indoxyl sulfate antibodies is not particularly limited, and examples include a tris buffer solution, phosphate buffered saline (PBS), a MES buffer solution, a MOPS buffer solution, a MOPSO buffer solution, a HEPES buffer solution, a TES buffer solution, a Tricine buffer solution, a Bis-Tris buffer solution, an ADA buffer solution, an ACES buffer solution, a PIPES buffer solution, a BES buffer solution, a DIPSO buffer solution, a TAPSO buffer solution, a POPSO buffer solution, a HEPPS buffer solution, a HEPPSO buffer solution, a Bicine buffer solution, a TAPS buffer solution, a CHES buffer solution, a CAPS buffer solution, an Imidazole-HCl buffer solution, a Glycylglycine buffer solution, or a Glycinamide buffer solution. A tris buffer solution is particularly preferable. The tris buffer solution is not particularly limited, and examples include a tris HCl buffer solution, a TE buffer solution, a TAE buffer solution, a TBE buffer solution, or tris buffered saline. Anti-indoxyl sulfate antibodies are dissolved in these buffer solutions to a concentration of 100 ng to 10 µg/mL and immobilized at 4°C and O/N at a volume of 50 µL to 200 µL/well.
As described above, the immobilized insoluble support includes cases in which anti-indoxyl sulfate antibodies are immobilized on an insoluble carrier via a component with affinity to the anti-indoxyl sulfate antibodies (for example, antibodies against the anti-indoxyl sulfate antibodies) or cases in which anti-indoxyl sulfate antibodies are immobilized on an insoluble support via the bonding of avidin, biotin, and the like. The immobilization of antibodies against the anti-indoxyl sulfate antibodies can be performed in the same manner as the immobilization of anti-indoxyl sulfate antibodies described above. In addition, the immobilization of avidin to the insoluble support and the biotin labeling of the anti-indoxyl sulfate antibodies can be performed in accordance with conventional methods.

The blocking step (2) is a step in which blocking is performed on the surface of the insoluble support by bringing a blocking solution into contact with the insoluble support in which anti-indoxyl sulfate antibodies are immobilized. The blocking solution is not limited as long as it contains substantially no mammalian albumin. Specific examples include a tris buffer solution, phosphate buffered saline (PBS), a MES buffer solution, a MOPS buffer solution, a MOPSO buffer solution, a HEPES buffer solution, a TES buffer solution, a Tricine buffer solution, a Bis-Tris buffer solution, an ADA buffer solution, an ACES buffer solution, a PIPES buffer solution, a BES buffer solution, a DIPSO buffer solution, a TAPSO buffer solution, a POPSO buffer solution, a HEPPS buffer solution, a HEPPSO buffer solution, a Bicine buffer solution, a TAPS buffer solution, a CHES buffer solution, a CAPS buffer solution, an Imidazole-HCl buffer solution, a Glycylglycine buffer solution, or a Glycinamide buffer solution.
In addition, the blocking solution may contain proteins other than mammalian albumin. The proteins other than mammalian albumin are not limited, and gelatin, casein, ovalbumin, commercially available blocking agents, or the like, for example, can be used.
In addition, the blocking solution may contain a surfactant. The surfactant is not particularly limited, and examples include nonionic surfactants, anionic surfactants, cationic surfactants, and/or amphoteric ion surfactants. Nonionic surfactants that do not inhibit antigen/antibody reactions are preferable, and Triton X, Tween, or Pluronic is particularly preferable.
In addition, the concentration of the surfactant is not particularly limited but is preferably from 0.002 to 2 wt.%, more preferably from 0.008 to 0.5 wt.%, and most preferably from 0.02 to 0.2 wt.%.
In the case of a 96-well plate, for example, blocking can be performed by adding such a blocking solution in an amount of 50 µL to 300 µL and letting the mixture stand for between 10 minutes and one day/night at 4°C to 37°C, for example.

### [4] Indoxyl sulfate measurement kit

The indoxyl sulfate measurement kit of the present invention includes (1) the immobilized insoluble support (A) or the immobilized insoluble support (B) described above and (2) a pretreatment solution for pretreating albumin in a specimen or a reagent for removing albumin in a specimen by deproteinization.

The immobilized insoluble support (A) and the immobilized insoluble support (B) may include those described in "[2] Immobilized insoluble support" above.

### (Pretreatment solution)

The pretreatment solution can be used in the indoxyl sulfate measurement method of the present invention and is capable of weakening bonds between indoxyl sulfate and mammalian albumin in the specimen and accelerating the binding of indoxyl sulfate and anti-indoxyl sulfate antibodies. A specific example is a buffer solution containing a surfactant. The surfactant is not limited, and examples include nonionic surfactants, anionic surfactants, cationic surfactants, and amphoteric ion surfactants. Nonionic Triton X, Tween, or Pluronic is particularly preferable.

In addition, the concentration of the surfactant is not particularly limited but is preferably from 0.002 to 2 wt.%, more preferably from 0.008 to 0.5 wt.%, and most preferably from 0.02 to 0.2 wt.%.

### (Deproteinization reagent)

The deproteinization reagent is not limited as long as it can substantially remove proteins including albumin without substantially removing indoxyl sulfate in the specimen, and known deproteinization reagents can be used without limitation. The principle of deproteinization treatment can use the method used in HPLC pretreatment, for example. Specific examples thereof include a method of modifying, insolubilizing, and removing proteins, a method of physically removing proteins by utilizing differences in molecule size, or an affinity method, but a method of modifying, insolubilizing, and removing proteins is preferable. This is because treatment in which proteins are insolubilized also has the effect of dissociating bonds between indoxyl sulfate and albumin. Commercially available reagents can also be used as the deproteinization reagent, and a Sirocco Protein Precipitation Plate, for example, may also be included in the kit of the present invention.

When measurement kit of the present invention includes the immobilized insoluble support (A), it includes anti-indoxyl sulfate antibodies. In addition, when the measurement kit includes the immobilized insoluble support (B), it includes labeled indoxyl sulfate. The anti-indoxyl sulfate antibodies or labeled indoxyl sulfate may be the anti-indoxyl sulfate antibodies or labeled indoxyl sulfate described in "[1] Indoxyl sulfate measurement method" above. Further, the measurement kit of the present invention may include an instruction manual clearly stating that it can specifically measure indoxyl sulfate in the blood. Such a description may also be appended to the container of the analysis kit.

### [5] Uremia diagnostic kit

The indoxyl sulfate measurement kit of the present invention can be used as a uremia diagnostic kit. Accordingly, the present invention relates to a uremia diagnostic kit including (1) the immobilized insoluble support (A) or the immobilized insoluble support (B) described above and (2) a pretreatment solution for pretreating albumin in a specimen or a reagent for removing albumin in a specimen by deproteinization. When the uremia diagnostic kit described above includes the immobilized insoluble support (A), it includes anti-indoxyl sulfate antibodies, and when the kit includes the immobilized insoluble support (B), it includes labeled indoxyl sulfate.

The indoxyl sulfate measurement kit of the present invention can be used for the diagnosis of uremia. Accordingly, the present invention relates to the use of an indoxyl sulfate measurement kit including (1) the immobilized insoluble support (A) or the immobilized insoluble support (B) described above and (2) a pretreatment solution for pretreating albumin in a specimen or a reagent for removing albumin in a specimen by deproteinization for the diagnosis of uremia. Further, when the indoxyl sulfate measurement kit described above includes the immobilized insoluble support (A), it includes anti-indoxyl sulfate antibodies, and when the kit includes the immobilized insoluble support (B), it includes labeled indoxyl sulfate.

Anti-indoxyl sulfate antibodies or labeled indoxyl sulfate can be used in the production of a uremia diagnostic kit (indoxyl sulfate measurement kit). Accordingly, the present invention relates to the use of anti-indoxyl sulfate antibodies or labeled indoxyl sulfate for the production of a uremia diagnostic kit (indoxyl sulfate measurement kit).
In addition, the immobilized insoluble support (A) or the immobilized insoluble support (B) can be used in the production of a uremia diagnostic kit (indoxyl sulfate measurement kit). Accordingly, the present invention relates to the use of an immobilized insoluble support in which indoxyl sulfate is immobilized and then blocked with a buffer solution containing substantially no mammalian albumin for the production of a uremia diagnostic kit (indoxyl sulfate measurement kit). Further, the present invention relates to the use of an immobilized insoluble support in which anti-indoxyl sulfate antibodies are immobilized and then blocked with a buffer solution containing substantially no mammalian albumin for the production of a uremia diagnostic kit (indoxyl sulfate measurement kit). Further, the pretreatment solution for pretreating albumin in a specimen or the reagent for removing albumin in a specimen by deproteinization can be used in the production of a uremia diagnostic kit (indoxyl sulfate measurement kit). Accordingly, the present invention relates to the use of a pretreatment solution for pretreating albumin in a specimen for the production of a uremia diagnostic kit (indoxyl sulfate measurement kit). Further, the present invention relates to the use of a reagent for removing albumin in a specimen by deproteinization for the production of a uremia diagnostic kit (indoxyl sulfate measurement kit).
Further, the present invention relates to the use of one or more selected from the group consisting of (1) anti-indoxyl sulfate antibodies, (2) an immobilized insoluble support in which indoxyl sulfate is immobilized and then blocked with a buffer solution containing substantially no mammalian albumin, and (3) a pretreatment solution for pretreating albumin in a specimen and/or a reagent for removing albumin in a specimen by deproteinization in the production of a uremia diagnostic kit. Further, the present invention relates to the use of one or more selected from the group consisting of (1) labeled indoxyl sulfate, (2) an immobilized insoluble support in which anti-indoxyl sulfate antibodies are immobilized and then blocked with a buffer solution containing substantially no mammalian albumin, and (3) a pretreatment solution for pretreating albumin in a specimen and/or a reagent for removing albumin in a specimen by deproteinization in the production of a uremia diagnostic kit.

### Examples

The present invention will be described in detail hereinafter using working examples, but these working examples do not limit the scope of the present invention.

### <Production Example 1: Preparation of linker-providing indoxyl sulfate>

Preparation of 1-(2',3'-epoxypropyl)indoxyl sulfate sodium First, 1.00 g of a commercially available indoxyl sulfate potassium salt was dissolved in 20 mL of dimethylformamide (called "DMF" hereafter), and 222 mg of sodium hydroxide (60%, oily substance) was then added and stirred for one hour at room temperature. Next, 934 µL of epichlorohydrin was added to the mixture and stirred for 5.5 hours at room temperature. After the reaction was complete, the reaction was ended by adding distilled water, and the solvent was then distilled out. Methanol was added to the residue, and insoluble matter was removed. The solvent of the filtrate was distilled out and purified by silica gel column chromatography (Kieselgel 60, 40 g, chloroform/methanol=10/1 to 10/2) to obtain 749 mg (64%) of compound (1) of the title as a light yellow foamy substance. MS (FAB., negative) m/z=268 [M-23]-1H-NMR(CDCl₃)δ (ppm): 2.59 (dd, 1H), 2.77 (dd, 1H), 3.17 (m, 1H), 4.11 (dd, 1H), 4.43 (dd, 1H), 6.96 (t,1H), 7.08(t, 1H), 7.16 (s, 1H), 7.44 (d, 1H), 7.50 (d, 1H).

<Production Example 2: Preparation of indoxyl sulfate and transferrin conjugate> The binding of the epoxy-form indoxyl sulfate obtained in Production Example 1 and transferrin (derived from human serum, Sigma Cat. T-2252) was performed for 96 hours at 4°C by introducing a sulfhydryl group into transferrin in advance using N-succimidyl-3-(2-pyridyldithio)propionate (Funakoshi Cat. No. FM-0185-31) and then mixing both substances at a molar ratio of 90:1 (58.5 mg to 160 mg/14 mL phosphate buffer solution, 0.1 M, pH 6.0, 2.5 mM EDTA). After the reaction, dialysis was performed for 24 hours at 4°C on Dulbecco's phosphate buffered saline (containing no Ca⁺⁺Mg⁺⁺, pH 7.4), and the internal fluid was centrifuged (5000xg, 10 minutes) to remove insoluble matter. The amount of binding to proteins was estimated to be two molecules of transferrin. The estimation of the number of bonds was performed by means of calculation from a 275 nm increase in absorption per 1 mg/mL of protein. The assay of proteins was performed with a dye method (protein assay kit, Japan Bio-Rad Laboratories, Cat. No. 500-0001), and 275 nm was calculated using a value of 0.9 as the value for 1 mg/mL in the case of transferrin and 20 in the case of the epoxy form indoxyl sulfate.

<Production Example 3: Preparation of anti-indoxyl sulfate mouse monoclonal antibodies> The indoxyl sulfate antigen (transferrin-IS) prepared in Production Example 2 was mixed with an equal amount of Freund's complete adjuvant, and 10 µg was intraperitoneally administered to BALB/c mice 4 to 6 weeks of age in terms of indoxyl sulfate content. Additional immunization was performed with 10 µg of Freund's incomplete adjuvant one week later, and two additional immunizations were repeated.

On day 3 after the final immunization, the spleen was aseptically extracted from the mouse, and the spleen was loosened into individual cells using scissors and forceps and washed three times with an RPMI-1640 medium. After the mouse myeloma cell line P3U1 in a logarithmic growth phase was washed three times with an RPMI-1640 medium, the cells and spleen cells were mixed at a cell count ratio of 1:10. After centrifugation for five minutes at 200xg, the supernatant was removed, and 1 mL of 50% polyethylene glycol (PEG) 1500 (Roche) was added while slowly mixing a cell aggregation. 9 mL of an RPMI-1640 medium was further added to achieve cell fusion.

After PEG was removed by centrifugation (200xg, 5 minutes), the fused cells were suspended in an RPMI-1640 medium containing 10% fetal bovine serum, hypoxanthine, aminopterin, and thymidine (HAT) and seeded in a 96-well cell culturing plate. After this was cultured for seven days to grow only hybridomas, clones producing antibodies were retrieved by an ELISA method so as to obtain a hybridoma producing monoclonal antibodies having the target reaction specificity.
The resulting hybridoma was prepared as a single clone by limiting dilution so as to establish an antibody-producing hybridoma (9A2F6). The isotype of the resulting monoclonal antibodies (9A2F6) was IgG2b.

### <Determination of the base sequence of the variable region genes of monoclonal antibodies>

Total RNA was extracted by a conventional method from the hybridoma producing the 9A2F6 antibodies, and a reverse transcription reaction was performed using an oligo-dT primer to produce cDNA. PCR was performed using a mouse Ig primer set (Novagen, Inc.) in accordance with the protocol thereof in order to amplify the variable region genes from the resulting cDNA. The resulting antibody variable region genes were TA-cloned into a pCR2.1 vector to determine the base sequence. The base sequence of the light chain variable region domain of 9A2F6 is shown in SEQ ID NO. 1; the amino acid sequence thereof is shown in SEQ ID NO. 2; the base sequence of nucleotides of the heavy chain variable region domain is shown in SEQ ID NO. 3; and the amino acid sequence thereof is shown in SEQ ID NO. 4.

### (Comparative Example 1)

In this comparative example, 9A2F6 described above was used, and the recovery rate was investigated after indoxyl sulfate was added to serum. Although albumin in the specimen was not pretreated, a reaction buffer solution containing no exogenous mammalian albumin and a blocking solution containing no mammalian albumin were used.

### (a) Preparation of indoxyl sulfate-containing specimens

Specimens for measurement were prepared by adding indoxyl sulfate to rat serum and human serum. Indoxyl sulfate (Alfer Aeser Inc.) was diluted with a tris buffer solution and added to rat serum and human serum to form a concentration of 31.3 µg/mL, 15.6 µg/mL, 7.81 µg/mL, 3.90 µg/mL, 1.95 µg/mL, 0.98 µg/mL, or 0.49 µg/mL.

### (b) Immobilized plate production

The transferrin-bound indoxyl sulfate (also called "Tf-IS" hereafter) obtained in Production Example 2 was diluted to 1 µg/mL with a tris buffer solution. This was added to an ELISA plate (IWASKI Inc.) at 50 ng/50 µL/well and left to stand at 4°C and O/N to immobilize the indoxyl sulfate. The solid phase solution was removed and washed three times with a washing solution (0.05% Tween 20 in TBS). Next, 300 µL of a blocking solution (Trisma base) was added, and blocking was performed for 30 minutes at 25°C.

### (c) Indoxyl sulfate measurement

First, 50 µL of the indoxyl sulfate-containing specimens described above (rat serum and human serum) and 50 µL of 9A2F6 monoclonal antibodies diluted to 1 µg/mL with a reaction buffer solution (0.05% Tween 20 in TBS) were mixed, and the mixture was reacted for 90 minutes at 37°C. The blocking solution of the immobilized plate was removed, and 50 µL of the mixture was added to the wells and reacted for 45 minutes at 37°C. After this was washed three times with a washing solution, 50 µL of anti-mouse IgG rabbit IgGFab'-HRP diluted to 0.1 µg/mL with a detection reaction buffer solution (0.05% Tween 20 in TBS) was added and reacted for 45 minutes at 37°C. After this was washed three times with a washing solution, 50 µL of a chromogenic substrate solution (TMB) was added and developed for 15 minutes at room temperature. The reaction was then stopped by adding 50 µL of a reaction stopping solution (IN H₂SO₄). The absorbance was measured with a plate reader (iMark, Bio-Rad). The results are shown in Table 1.

**[Table 1]**

| Theoretical value (µg/mL) | Rat Serum | Human Serum |
|---|---|---|
| | Recovery rate (%) | Recovery rate (%) |
| 31.3 | 32.9 | 10.8 |
| 15.6 | 10.3 | 0.9 |
| 7.81 | 0.0 | 0.0 |
| 3.90 | 0.0 | 0.0 |
| 1.95 | 0.0 | 0.0 |
| 0.98 | 0.0 | 0.0 |
| 0.49 | 0.0 | 0.0 |

Even when 31.3 µg/mL of indoxyl sulfate was added, the recovery rates were extremely low at 32.9% and 10.8%, respectively, in rat serum and human serum. In addition, measurements were not possible at all at 7.81 µg/mL or lower.

### (Reference Example 1)

In this reference example, a buffer solution was used instead of human serum to investigate the effects when BSA was added to a blocking solution, a reaction buffer solution, and a detection reaction buffer solution.

### (Preparation of indoxyl sulfate-containing specimens)

Indoxyl sulfate (Alfer Aeser Inc.) was diluted two times to 500 µg/mL to 61 ng/mL with a tris buffer solution.

### (Immobilized plate production)

The transferrin-bound Tf-IS obtained in Production Example 2 was diluted to 1 µg/mL with Trizma-base (SIGMA). This was added to an ELISA plate (IWAKI Inc.) at 50 ng/50 µL/well and left to stand at 4°C and O/N to immobilize the indoxyl sulfate. The solid phase solution was removed and washed three times with a washing solution (0.05% Tween 20 in TBS). A blocking solution to which 300 µL BSA was not added (Trisma base) or a blocking solution to which BSA was added (1% BSA in PBS) was added, and blocking was performed for 30 minutes at 25°C.

### (Indoxyl sulfate measurement)

First, 50 µL of the indoxyl sulfate-containing specimens described above and 50 µL of 9A2F6 monoclonal antibodies or 22-40-B5 monoclonal antibodies diluted to 1 µg/mL with a reaction buffer solution to which BSA was added (1% BSA, 0.05% Tween 20 in TBS) or a reaction buffer solution to which BSA was not added (0.05% Tween 20 in TBS) were mixed, and the mixture was reacted for 90 minutes at 37°C. The blocking solution of the immobilized plate was removed, and 50 µL of the mixture was added to the wells and reacted for 45 minutes at 37°C. After this was washed three times, 50 µL of anti-mouse IgG rabbit IgGFab'-HRP diluted to 0.1 µg/mL with a reaction buffer solution to which BSA was added (1% BSA, 0.05% Tween 20 in TBS) or a detection reaction buffer solution to which BSA was not added (0.05% Tween 20 in TBS) was added and reacted for 45 minutes at 37°C. After this was washed three times with a washing solution, 50 µL of a chromogenic substrate solution (TMB) was added and developed for 15 minutes at room temperature. The reaction was then stopped by adding 50 µL of a reaction stopping solution (IN H₂SO₄). The absorbance was measured with a plate reader (iMark, Bio-Rad). The results are shown in FIG. 1.

In the case of 22-40-B5 monoclonal antibodies, the sensitivity was 15.6 µg/mL when a blocking solution, reaction buffer solution, and detection reaction buffer solution to which BSA was added was used. On the other hand, the sensitivity increased to 3.9 µg/mL when a blocking solution, reaction buffer solution, and detection reaction buffer solution to which BSA was not added was used.
In addition, in comparison to 22-40-B5 monoclonal antibodies, the sensitivity increased to 0.49 µg/mL when 9A2F6 monoclonal antibodies were used.

It became clear from the aforementioned Comparative Example 1 and Reference Example 1 that the blocking solution, reaction buffer solution, and detection reaction buffer solution to which BSA was added inhibited the measurement of indoxyl sulfate and that the measurement of indoxyl sulfate is difficult when rat serum and human serum are used.

### (Working Example 1)

In this working example, human serum was used, and measurements were performed using a blocking solution, reaction buffer solution, and detection reaction buffer solution to which BSA was not added without subjecting the specimens to deproteinization treatment. Specimens for measurement were prepared by adding indoxyl sulfate to human serum. Indoxyl sulfate (Alfer Aeser Inc.) was diluted with a tris buffer solution and added to human serum to form a concentration of 25.0 µg/mL, 12.5 µg/mL, 6.25 µg/mL, 3.13 µg/mL, 0.78 µg/mL, 0.39 µg/mL, or 0.20 µg/mL.

### (Treatment with a pretreatment solution)

Treatment with a pretreatment solution was performed by adding a pretreatment solution of at least double volume (Immunobiological Laboratories Co., Ltd.) to human serum.

### (Indoxyl sulfate measurement)

The operations of "(c) Indoxyl sulfate measurement" of Comparative Example 1 described above were repeated with the exception that specimens treated with a pretreatment solution were used. The results are shown in Table 2.

**[Table 2]**

| Theoretical value (µg/mL) | Human Serum |
|---|---|
| | Recovery rate (%) |
| 31.3 | 279.2 |
| 15.6 | 140.2 |
| 7.81 | 103.9 |
| 3.90 | 93.4 |
| 1.95 | 29.5 |
| 0.98 | 0.0 |
| 0.49 | 0.0 |

### (Working Example 2)

In this working example, rat serum and human serum were used, and the specimens were subjected to deproteinization treatment. Further, measurements were performed using a blocking solution, reaction buffer solution, and detection reaction buffer solution to which BSA was not added.

### (Preparation of indoxyl sulfate-containing specimens)

Specimens for measurement were prepared by adding indoxyl sulfate to rat serum and human serum. Indoxyl sulfate (Alfer Aeser Inc.) was diluted with a tris buffer solution and added to rat serum and human serum to form a concentration of 25.0 µg/mL, 12.5 µg/mL, 6.25 µg/mL, 3.13 µg/mL, 0.78 µg/mL, 0.39 µg/mL, or 0.20 µg/mL.

### (Deproteinization)

Deproteinization was performed using a Sirocco Protein Precipitation Plate. After 200 µL of acetonitrile was added to the plate, 50 µL of rat serum or human serum to which indoxyl sulfate was added was added. This was stirred for one minute, and after it was aspirated with a manifold, the filtrate was recovered and cryopreserved.

### (Indoxyl sulfate measurement)

The operations of "(c) Indoxyl sulfate measurement" of Comparative Example 1 described above were repeated with the exception that specimens subjected to deproteinization were used. The results are shown in Table 3.

**[Table 3]**

| Theoretical value (µg/mL) | Rat Serum | Human Serum |
|---|---|---|
| | Recovery rate (%) | Recovery rate (%) |
| 25.0 | 109.4 | 96.4 |
| 12.5 | 128.6 | 134.1 |
| 6.25 | 110.1 | 109.8 |
| 3.13 | 117.7 | 124.2 |
| 0.78 | 122.1 | 98.1 |
| 0.39 | 121.2 | 89.2 |
| 0.20 | 84.3 | 89.3 |

In the cases of both rat serum and human serum, it was possible to recover approximately 100% of the indoxyl sulfate by performing deproteinization. In addition, it was also possible to improve the sensitivity to 0.2 µg/mL.

### Industrial Applicability

The indoxyl sulfate measurement method of the present invention can accurately measure indoxyl sulfate, which is a uremic toxin in the blood of patients with diseases to which indoxyl sulfate contributes (for example, renal disorder patients) and can be used in the diagnosis or treatment monitoring of diseases to which indoxyl sulfate contributes (for example, renal disorder). The present invention was described above using specific modes of embodiment, but modifications and improvements apparent to persons having ordinary skill in the art are also included in the scope of the present invention.

## Claims

1. An indoxyl sulfate measurement method based on a competitive method comprising:
(1) a step in which albumin in a specimen is pretreated;
(2) a specimen-antibody reaction step in which the pretreated specimen and anti-indoxyl sulfate antibodies are brought into contact with each other in the substantial absence of exogenous mammalian albumin;
(3) an immobilized IS-antibody reaction step in which the specimen-antibody reaction solution is brought into contact, in the substantial absence of exogenous mammalian albumin, with an immobilized insoluble support in which indoxyl sulfate is immobilized and then blocked with a buffer solution containing substantially no mammalian albumin; and
(4) a step in which anti-indoxyl sulfate antibodies which have bonded to the immobilized insoluble support are detected.

2. An indoxyl sulfate measurement method based on a competitive method comprising:
(1) a step in which albumin in a specimen is pretreated;
(2) a specimen-antigen reaction step in which the pretreated specimen and labeled indoxyl sulfate are brought into contact with each other in the substantial absence of exogenous mammalian albumin;
(3) an immobilized antibody-labeled IS reaction step in which the specimen-antigen reaction solution is brought into contact, in the substantial absence of exogenous mammalian albumin, with an immobilized insoluble support in which anti-indoxyl sulfate antibodies are immobilized and then blocked with a buffer solution containing substantially no mammalian albumin; and
(4) a step in which labeled indoxyl sulfate which has bonded to the immobilized insoluble support is detected.

3. The indoxyl sulfate measurement method according to one of claim 1 or 2, wherein the anti-indoxyl sulfate antibodies are antibodies containing a light chain complementarity determining region comprising an amino acid sequence represented by SEQ ID NO: 2 and a heavy chain complementarity determining region comprising an amino acid sequence represented by SEQ ID NO: 4.

4. The indoxyl sulfate measurement method according to one of claims 1 to 3, wherein the pretreatment of albumin is treatment with a pretreatment solution or deproteinization treatment.

5. The indoxyl sulfate measurement method according to one of claims 1, 3, and 4, wherein the specimen-antibody reaction step (2) and the immobilized IS-antibody reaction step (3) are performed simultaneously.

6. The indoxyl sulfate measurement method according to one of claims 2 to 4, wherein the specimen-antigen reaction step (2) and the immobilized antibody-labeled IS reaction step (3) are performed simultaneously.

7. An immobilized insoluble support in which indoxyl sulfate is immobilized and then blocked with a buffer solution containing substantially no mammalian albumin.

8. An immobilized insoluble support in which anti-indoxyl sulfate antibodies are immobilized and then blocked with a buffer solution containing substantially no mammalian albumin.

9. An indoxyl sulfate measurement kit including:
(1) the immobilized insoluble support according to claim 7 or the immobilized insoluble support according to claim 8; and
(2) a pretreatment solution for pretreating albumin in a specimen or a reagent for removing albumin in a specimen by deproteinization.

10. An anti-indoxyl sulfate antibody containing a light chain complementarity determining region comprising an amino acid sequence represented by SEQ ID NO: 2 and a heavy chain complementarity determining region comprising an amino acid sequence represented by SEQ ID NO: 4.
